Europäisches Patentamt

(19) European Patent Office

Office européen des brevets

(11) Publication number: **0 297 211 B1**

# (12) EUROPEAN PATENT SPECIFICATION

(45) Date of publication of patent specification:
28.08.91 Bulletin 91/35

(51) Int. Cl.⁵: **A61K 7/00, A61K 7/16, A61K 7/18**

(21) Application number: 88103994.5

(22) Date of filing: 14.06.83

(54) Oral compositions.

(30) Priority: 22.06.82 US 391040

(43) Date of publication of application:
04.01.89 Bulletin 89/01

(45) Publication of the grant of the patent:
28.08.91 Bulletin 91/35

(84) Designated Contracting States:
**AT BE CH DE FR GB IT LI NL SE**

(56) References cited:
US-A- 2 876 167
US-A- 3 137 632
US-A- 3 608 068
US-A- 3 678 154
US-A- 3 927 202
US-A- 4 244 931
ARCH. ORAL BIOL., vol. 15, 1970, Pergamon Press, GB; F.J. DRAUS et al.: "Pyrophosphate and hexametaphosphate effects in in vitro calculus formation", pages 893-896

(60) Publication number of the earlier application in accordance with Art. 76 EPC: **0 097 476**

(73) Proprietor: **THE PROCTER & GAMBLE COMPANY**
**One Procter & Gamble Plaza**
**Cincinnati Ohio 45202 (US)**

(72) Inventor: **Parran, John Joseph, Jr.**
**1155 Meredith Drive**
**Cincinnati Ohio 45231 (US)**
Inventor: **Sakkab, Nabil Yaqub**
**8297 Glenmill Road**
**Cincinnati Ohio 45249 (US)**

(74) Representative: **Brooks, Maxim Courtney et al**
**Procter & Gamble (NTC) Limited Whitley Road Longbenton**
**Newcastle-upon-Tyne NE12 9TS (GB)**

## Description

The present invention relates to oral compositions in the form of toothpastes which provide an anticalculus benefit.

Dental calculus, or tartar as it is sometimes called, is a deposit which forms on the surfaces of the teeth at the gingival margin. Supragingival calculus appears principally in the areas near the orifices of the salivary ducts ; e.g., on the lingual surfaces of the lower anterior teeth and on the buccal surfaces of the upper first and second molars, and on the distal surfaces of the posterior molars.

Mature calculus consists of an inorganic portion which is largely calcium phosphate arranged in a hydroxyapatite crystal lattice structure similar to bone, enamel and dentine. An organic portion is also present and consists of desquamated epithelial cells, leukocytes, salivary sediment, food debris and various types of microorganisms.

As the mature calculus develops, it becomes visibly white or yellowish in color unless stained or discolored by some extraneous agent. In addition to being unsightly and undesirable from an aesthetic standpoint, the mature calculus deposits are constant sources of irritation of the gingiva.

A wide variety of chemical and biological agents have been suggested in the art to retard calculus formation or to remove calculus after it is formed. Mechanical removal of this material periodically by the dentist is, of course, routine dental office procedure.

The chemical approach to calculus inhibition generally involves chelation of calcium ion and/or crystal growth inhibition which prevents the calculus from forming and/or breaks down mature calculus by removing calcium.

The prior art discloses a number of chelating agents for this purpose. GB-A-490,384, February 15, 1937, discloses oral compositions containing ethylenediaminetetraacetic acid, nitrilotriacetic acid and related compounds as anticalculus agents. US-A-3,678,154 July 18, 1972 to Widder et al discloses oral compositions containing certain polyphosphonates and fluoride. US-A-3,737,533, June 5, 1973 to Francis discloses oral compositions containing certain carbonyl diphosphonates.

In addition to the above references, the prior art discloses dentifrices and mouthwashes containing soluble pyrophosphate salts which have been indicated for a variety of purposes. Included among such references are US-A-2,941,926, June 21, 1960 to Salzmann et al which discloses dental powders containing chlorophyll and pyrophosphate salts. US-A-3,137,632, June 16, 1964 to Schiraldi discloses toothpastes containing pyrophosphate salts. US-A-3,927,201 and US-A-3,927,202, December 16, 1975 to Baines et al and Harvey et al, respectively discloses toothpastes which utilize soluble pyrophosphates as abrasives. US-A-4,244,931, January 13, 1981 and US-A-4,247,526, January 27, 1981 to Jarvis et al disclose pyrophosphate salts in dicalcium phosphate systems. Jap. Patent Application Disclosure No. 4945-1974 discloses soluble pyrophosphates in a variety of dentifrice systems. US-A-4,323,551, April 6, 1982 to Parran discloses tetraalkali metal salts in mouthwash compositions, Finally Draus, Lesniewski and Miklos, Pyrophosphate and Hexametaphosphate Effects In Vitro Calculus Formation, Arch. Oral Biol., Vol. 15, pp. 893-896, (1970) disclose the in vitro effectiveness of soluble pyrophosphate salts against calculus. However, they indicate that pyrophosphate would be inhibited by pyrophosphatase in vivo.

In spite of the many disclosures in the anticalculus and pyrophosphate areas, the need for an effective anticalculus product still exists. Surprisingly mixtures of certain pyrophosphate salts can provide a safe and effective product while also not presenting difficult formulation problems.

It is an object of the present invention to provide toothpaste compositions which deliver an effective aticalculus benefit.

It is a further object of the present invention to provide an effective anticalculus toothpaste product utilizing a mixture of soluble pyrophosphate salts.

These and other objects will become more clear from the detailed description which follows.

All percentages and ratios used herein are by weight unless otherwise specified.

The present invention relates to an oral composition in the form of a toothpaste comprising :

a) from 10% to 70% by weight of a dental abrasive ;

b) an amount of a fluoride ion source capable of providing from 50 ppm to 3500 ppm of fluoride ions ;

c) an amount of a pyrophosphate salt selected from dialkali metal and mixtures of dialkali metal and tetraalkali metal pyrophosphate salts sufficient to provide at least 1.5% by weight of pyrophosphate ions $(P_2O_7^{-4})$ ; and

d) water ;

wherein the pH of the composition is from 6.0 to 10.0 and the composition does not contain more than 4.0% by weight of tetrapotassium pyrophosphate ($K_4P_2O_7$) and wherein are excluded compositions wherein the dental abrasive is selected from insoluble metaphosphates, alumina, thermosetting polymerized resins, silica and mixtures thereof.

The essential as well as optional components of the compositions of the present invention are described in the following paragraphs :

## Dental Abrasive

The abrasives useful in the dentifrice composition aspect of the present invention include many different materials. Calcium pyrophosphate, including the $\beta$-phase calcium pyrophosphate prepared in accordance with the teaching of Schweizer, US-A-3,112,247, Nov. 26, 1963, can be used. The $\beta$-phase calcium pyrophosphate is prepared by heating $\gamma$-phase calcium pyrophosphate to 700-900°C. to change at least 50% of the $\gamma$-phase to $\beta$-phase and then immediately cooling.

The total amount of abrasive in the dentifrice embodiments of this invention ranges from 10% to 70% by weight of the dentifrice. Preferably, toothpastes contain from 10% to 50% by weight of abrasive.

## Fluoride Ion Source

The second essential component of the compositions herein is a fluoride ion source, The number of such sources is great and includes those disclosed in US-A-3,535,421, October 20, 1970 to Briner et al. Typical materials include :

Stannous fluoride, potassium fluoride, lithium fluoride, cesium fluoride, ammonium fluoride, aluminum fluoride, cupric fluoride, indium fluoride, stannous fluorozirconate, lead fluoride, ferric fluoride, nickel fluoride, paladium fluoride, silver fluoride, zinc fluoride, zirconium fluoride, hexylamine hydrofluoride, laurylamine hydrofluoride, myristylamine hydrofluoride, decanolamine hydrofluoride, octadecenylamine hydrofluoride, myristoxyamine, hydrofluoride, diethylaminoethyloctoylamide hydrofluoride, diethanolamineoethyloleylamide hydrofluoride, diethanolaminopropyl-N'-octadecenylamine dihydrofluoride, 1-ethanol-2-hexadecylimidazoline dihydrofluoride, octoylethanolamine hydrofluoride, octyltrimethylammonium fluoride, dodecylethyldimethylammonium fluoride, tetraethylammonium fluoride, dilauryldimethylammonium fluoride. $\Delta^{8,9}$-octadecenylbenzyldimethylammonium fluoride, dioctyldiethylammonium fluoride, cyclohexylcetyldimethylammonium fluoride, furfuryllauryldimethylammonium fluoride, phenoxyethylcetyldimethylammonium fluoride, N :N'-tetramethyl-N :N ;-dilaurylethylenediammonium difluoride, N-cetylpyridinium fluoride, N :N-dilauryl-morpholinium fluoride, N-myristyl-N-ethylmorpholinium fluoride, N-(octylaminocarbonylethyl)-N-benzyl-dimethylammonium fluoride, N($\beta$-hydroxydodecyl) trimethylammonium fluoride, N-phenyl-N-hexadecyldiethylammonium fluoride, N-cyclohexyl-N-octadecyldimethylammonium fluoride, N-(2-carbomethoxyethyl)-N-benzyldimethylammonium fluoride, N-(2-carbocyclohexoxyethyl) N-myristyldimethylammonium fluoride, N-(2-carbobenzyloxyethyl)-N-dodecyldimethylammonium fluoride, N-[2-(N :N'-dimethylaminocarbonyl)-ethyl]-N-dodecyldiethylammonium fluoride, N-carboxymethyl-N-cicosyldimethylammonium fluoride, betaine hydrofluoride, sarcosine stannous fluoride, alanine stannous fluoride, glycine potassium fluoride, sarcosine potassium fluoride, glycine hydrofluoride, lysine hydrofluoride, alanine hydrofluoride, betaine zirconium fluoride, sodium monofluoro phosphate and mixtures thereof. Sodium fluoride is the preferred fluoride source.

The amount of the fluoride ion source should be sufficient to provide from 50 ppm to 3500 ppm, preferably from 500 ppm to 3000 ppm of fluoride ions.

## Di alkali Metal and Tetra alkali Metal Salts

The pyrophosphate salts useful in the present compositions are selected from dialkali metal pyrophosphate and mixtures of the dialkali metal and tetraalkali metal pyrophosphate salts. $Na_2H_2P_2O_7$, $Na_4P_2O_7$ and $K_4P_2O_7$ in their unhydrated as well as hydrated forms are the preferred species. The levels of each of these species which preferably are used in the compositions are as follows (all are in the unhydrated form).

$$Na_2H_2P_2O_7 \quad - \quad 0.5\% \quad - \quad 13.8\%$$
$$Na_4P_2O_7 \quad - \quad 0 \quad - \quad 6.0\%$$
$$K_4P_2O_7 \quad - \quad 0 \quad - \quad 4.0\%$$

The minimum amount of $P_2O_7{}^{-4}$ required in the present compositions, 1.5% is therefore provided solely by

$Na_2H_2P_2O_7$ or mixtures of $Na_2H_2P_2O_7$ with either or both of the tetra alkali metal salts. Preferred are binary mixtures of the sodium salts and ternary mixtures of those with the tetra potassium salt. The upper limits on the sodium species are determined by solubility considerations while the tetra potassium level is established for taste reasons.

The pyrophosphate salts are described in more detail in Kirk & Othmer, Encyclopedia of Chemical Technology, Second Edition, Volume 15, Interscience Publishers (1968).

Water

Water is another essential component of the compositions of this invention. Water employed in the preparation of commercially suitable oral compositions should preferably be of low ion content and free of organic impurities. Water preferably comprises from 2% to 95%, more preferably from 2% to 45% of the compositions of this invention.

Optional Components

In addition to the above described essential components, the oral compositions of this invention can contain a variety of optional conventional oral composition components. Such optional ingredients include sudsing agents, flavoring agents, sweetening agents, antiplaque agents, coloring agents, and pigments.

A preferred optional ingredient is a sudsing agent. Suitable sudsing agents are those which are reasonably stable and form suds throughout a wide pH range, i.e., non-soap anionic, nonionic, cationic, zwitterionic and amphoteric organic synthetic detergents. Sudsing agents of these types are described more fully in Agricola et al ; US-A-3,959,458 ; issued May 25, 1976 and in Haefele ; US-A-3,937,807 ; issued February 10, 1976.

Anionic sudsing agents useful herein include the water-soluble salts of alkyl sulfates having from 10 to 18 carbon atoms in the alkyl radical and the water-soluble salts of sulfonated monoglycerides of fatty acids having from 10 to 18 carbon atoms. Sodium lauryl sulfate and sodium coconut monoglyceride sulfonates are examples of anionic surfactants of this type. Mixtures of anionic surfactants can also be employed.

The nonionic sudsing agents which can be used in the compositions of the present invention can be broadly defined as compounds produced by the condensation of alkylene oxide groups (hydrophilic in nature) with an organic hydrophobic compound which may be aliphatic or alkylaromatic in nature. Examples of suitable nonionic sudsing agents include the Pluronics (RTm), polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

The zwitterionic synthetic sudsing agents useful in the compositions of the present invention can be broadly described as derivatives of aliphatic quaternary ammonium, phosphonium, and sulfonium compounds, in which the aliphatic radicals can be straight chain or branched, and wherein one of the aliphatic substituents contains from about 8 to 18 carbon atoms and one contains an anionic watersolubilizing group, e.g., carboxy, sulfonate, sulfate, phosphate, orphosphonate.

The cationic sudsing agents useful in the compositions of the present invention can be broadly defined as quaternary ammonium compounds having one long alkyl chain containing from about 8 to about 18 carbon atoms such as lauryl trimethylammonium chloride ; cetyl pyridinium chloride, cetyl trimethylammonium bromide ; di-isobutylphenoxyethoxyethyl-dimethylbenzylammonium chloride ; coconutalkyltrimethylammonium nitrite ; cetyl pyridinium fluoride ; etc.

The amphoteric sudsing agents useful in the present invention can be broadly described as derivatives of aliphatic secondary and tertiary amines in which the aliphatic radical can be straight chain or branched and wherein one of the aliphatic substituents contains from about 8 to about 18 carbon atoms and one contains an anionic water-solubilizing group, e.g., carboxylate, sulfonate, sulfate, phosphate, or phosphonate.

The sudsing agent can be present in the compositions of this invention in an amount from 0% to 10% by weight of the total composition.

Flavoring agents can also be added to the instant compositions. Suitable flavoring agents include oil of wintergreen, oil of peppermint, oil of spearmint, oil of sassafras, and oil of clove. Sweetening agents which can be used include saccharin, dextrose, levulose, aspartame, D-tryptophan, dihydrochalcones, acesulfame and sodium cyclamate. Flavoring agents are generally used in the compositions at levels of from 0.4% to 2% by weight and sweetening agents at levels of from 0.1% to 5% by weight.

Binders can also be used with the toothpastes of of the present inventions. Such binders include, for example, xanthan gum, carrageenan (Irish moss, Viscarin (RTm), and carboxyvinyl polymers. These binders are generally present at a level of from 0.1% to 1%.

Bis-biguanide antiplaque agents can also optionally be added to the compositions of this invention. Such agents include chlorhexidine (1,6-bis [$N^5$-p-chlorophenyl-$N^1$-biguanido]hexane), the soluble and insoluble salts thereof and related materials such as 1,2-bis($N^5$-p-trifluoromethylphenyl-$N^1$-biguanido)ethane are described more fully in US-A-3,923,002, US-A-3,937,807, BE-A-843,244, and BE-A-844,764.

If present, the optional antiplaque agents generally comprise from 0% to 5% by weight of the compositions herein.

Another optional component of the compositions herein is a humectant. The humectant serves to keep the toothpaste compositions from hardening upon exposure to air and in mouthwashes give a moist feel to the mouth. Certain humectants can also impart desirable sweetness of flavor to toothpaste compositions. The humectant, on a pure humectant basis, generally comprises from 0% to 70%, preferably from 0% to 55%, by weight of the compositions herein.

Suitable humectants for use in this invention include edible polyhydric alcohols such as glycerine, sorbitol, xylitol and propylene glycol. Sorbitol is frequently employed as a 70% aqueous solution known as Sorbo (RTtm).

The pH of the compositions herein is in the range of 6,0 to 10.0, preferably from 7.3 to 9.0. The pH is preferably achieved through a proper balancing of the pyrophosphate salts or by the addition of an alkaline or acidic agent.

## Claims

1. An oral composition in the form of a toothpaste comprising :

a)    from 10% to 70% by weight of a dental abrasive ;

b)    an amount of a fluoride ion source capable of providing from 50 ppm to 3500 ppm of fluoride ions ;

c)    an amount of a pyrophosphate salt selected from dialkali metal and mixtures of dialkali metal and tetraalkali metal pyrophosphate salts sufficient to provide at least 1.5% by weight of pyrophosphate ions ($P_2O_7^{-4}$ ;) and

d)    water ;

wherein the pH of said composition is from 6.0 to 10.0 and the composition does not contain more than 4.0% by weight of tetrapotassium pyrophosphate ($K_4P_2O_7$), and wherein are excluded compositions wherein the dental abrasive is selected from insoluble metaphosphates, alumina, thermosetting polymerized resins, silica and mixtures thereof.

2. An oral composition according to Claim 1 wherein the fluoride ion source is sodium fluoride.

3. An oral composition according to Claim 1 or 2 wherein the pyrophosphate ion is provided by a mixture of disodium pyrophosphate and tetrasodium pyrophosphate.

4. An oral composition according to Claim 1 or 2 wherein the pyrophosphate ion is provided by a mixture of disodium pyrophosphate, tetrasodium pyrophosphate and tetrapotassium pyrophosphate.

5. An oral composition according to any of Claims 1 to 4 which in addition contains from 0 to 10% by weight of a sudsing agent.

6. An oral composition according to any of Claims 1 to 5 which in addition contains from 0.1% to 1.0% by weight of a binder.

7. An oral composition according to any of Claims 1 to 6 which in addition contains from 0 to 70% by weight of a humectant.

8. An oral composition according to any of Claims 1 to 7 wherein the dental abrasive is β-phase calcium pyrophosphate.

## Patentansprüche

1. Orale Zusammensetzung in Form einer Zahnpaste, umfassend :

a)    10 Gew.-% bis 70 Gew.-% eines Dentalschleifmittels ;

b)    eine Menge einer Fluoridionenquelle, welche fähig ist, 50 ppm bis 3500 ppm Fluoridionen zu liefern ;

c)    eine Menge eines Pyrophosphatsalzes, ausgewählt unter Dialkalimetall und Gemischen aus Dialkalimetall- und Tetraalkalimetallpyrophosphatsalzen, die ausreichend ist, um mindestens 1,5 Gew.-% an Pyrophosphationen ($P_2O_7^{-4}$) zu liefern, und

d)    Wasser,

worin der pH-Wert der gennanten Zusammensetzung 6,0 bis 10,0 beträgt und die Zusammensetzung nicht mehr als 4 Gew. -% an Tetrakaliumpyrophosphat ($K_4P_2O_7$) enthält, und worin Zusammensetzungen ausgeschlossen sind, bei welchen das Dentalschleifmittel unter unlöslichen Metaphosphaten, Aluminiumoxid, hitzegehärteten polymerisierten Harzen, Siliciumdioxid und Gemischen hievon ausgewählt ist.

2. Orale Zusammensetzung nach Anspruch 1, worin die Fluoridionenquelle Natriumfluorid ist.

3. Orale Zusammensetzung nach Anspruch 1 oder 2, worin das Pyrophosphation von einem Gemisch aus Dinatriumpyrophosphat und Tetranatriumpyrophosphat geliefert wird.

4. Orale Zusammenstzung nach Anspruch 1 oder 2, worin das Pyrophosphation von einem Gemisch aus Dinatriumpyrophosphat, Tetranatriumpyrophosphat und Tetrakaliumpyrophosphat geliefert wird.

5. Orale Zusammensetzung nach einem der Ansprüche 1 bis 4, welche zusätzlich 0 bis 10 Gew.-% eines schäumenden mittels enthält.

6. Orale Zusammensetzung nach einem der Ansprüche 1 bis 5, welche zusätzlich 0,1 Gew.-% bis 1,0 Gew.-% eines Bindemittels enthält.

7. Orale Zusammensetzung nach einem Ansprüche 1 bis 6, welche zusätzlich 0 bis 70 Gew.-% eines Feuchthaltemittels enthält.

8. Orale Zusammensetzung nach einem der Ansprüche 1 bis 7, worin das Dentalschleifmittel Calciumpyrohosphat in der β-Phase ist.

## Revendications

1. Composition orale sous la forme d'une pâte dentifrice comprenant :

a)    de 10 à 70% en poids d'un abrasif dentaire ;
b)    une quantité d'une source d'ions fluorure capable de fournir de 50 à 3500 ppm d'ions fluorure ;
c)    une quantité d'un sel de type pyrophosphate choisi parmi des pyrophosphates dialcalins et des mélanges de pyrophosphates dialcalins et tétra-alcalins, suffisante pour fournir au moins 1,5% en poids d'ions pyrophosphate ($P_2O_7^{-4}$) et
d)    de l'eau ;

le pH de ladite composition allant de 6,0 à 10,0 et la composition ne contenant pas plus de 4,0% en poids de pyrophosphate tétrapotassique ($K_4P_2O_7$), et étant exclues des compositions dans lesquelles l'abrasif dentaire est choisi parmi des métaphosphates insolubles, l'alumine, des résines polymérisées thermodurcissables, la silice et des mélanges de ceux-ci.

2. Composition orale selon la revendication 1, dans laquelle la source d'ions fluorure est le fluorure de sodium.

3. Composition orale selon la revendication 1 ou 2, dans laquelle l'ion pyrophosphate est fourni par un mélange de pyrophosphate disodique et pyrophosphate tétrasodique.

4. Composition orale selon la revendication 1 ou 2, dans laquelle l'ion pyrophosphate est fourni par un mélange de pyrophosphate disodique, pyrophosphate tétrasodique et pyrophosphate tétrapotassique.

5. Composition orale selon l'un quelconque des revendications 1 à 4, qui contient en outre de 0 à 10% en poids d'un agent moussant.

6. Composition orale selon l'un quelconque des revendications 1 à 5, qui contient en outre de 0,1 à 1,0% en poids d'un liant.

7. Composition orale selon l'un quelconque des revendications 1 à 6, qui contient en outre de 0 à 70% en poids d'un humectant.

8. Composition orale selon l'un quelconque des revendications 1 à 7, dans laquelle l'abrasif dentaire est le pyrophosphate de calcium en phase β.